# EUROPÄISCHE PATENTANMELDUNG

(11) **EP 1 013 286 A2**
(43) Veröffentlichungstag der Anmeldung: **28.06.2000**
(21) Anmeldenummer: 99122579.8
(22) Anmeldetag: 12.11.1999
(51) Int. Cl.: A61K 47/26, A61K 9/14, A61K 31/00

(54) **Vitaminpräparat, Verfahren zu seiner Herstellung und seine Verwendung**

(30) Priorität: 17.12.1998 DE 19858372
(71) Anmelder: Vitafood AG, 9001 St. Gallen (CH)
(72) Erfinder: Latzke, Karin, 9434 Au (CH)
(74) Vertreter: Grünecker, Kinkeldey, Stockmair & Schwanhäusser Anwaltssozietät

(57) **Zusammenfassung**

Die vorliegende Erfindung betrifft ein Vitaminpräparat, umfassend (a) eine Komponente aus (A) einem Trägermaterial aus mindestens einem Zucker oder einem Zuckerderivat in Form eines feinen Pulvers, auf das (B) mindestens ein Vitamin und/oder Spurenelement und/oder Pflanzenextrakt aufgebracht ist, und (b) mindestens ein Bindemittel in kristalliner oder agglomerierter Form, sowie ein Verfahren zu seiner Herstellung und die Verwendung dieses Vitaminpräparates als Nahrungsergänzungsmittel und/oder Arzneimittel.

## Beschreibung

Die vorliegende Erfindung betrifft ein Vitaminpräparat, ein Verfahren zu seiner Herstellung sowie seine Verwendung als Nahrungsergänzungsmittel oder als Arzneimittel.

Vitamine gehören zu den essentiellen Nahrungsbestandteilen; sie müssen dem Organismus zugeführt werden und sind im allgemeinen nicht durch andere Stoffe ersetzbar. Durch das Fehlen von Vitaminen in der Nahrung kommt es zu Erkrankungen bzw. Mangelerscheinungen. Die Ursachen einer ungenügenden Zufuhr an Vitaminen liegen unter anderem in einer Einschränkung der Nahrungsaufnahme (Diät), einem gesteigertem Vitaminbedarf, der beispielsweise durch Schwangerschaft, Stillzeit, ungewöhnliche körperliche Leistungen oder gewisse Erkrankungen hervorgerufen werden kann, oder Zerstören von Vitaminen beim Zubereiten von Speisen. Um auftretende Vitaminverluste auszugleichen, werden verschiedene Lebensmittel, wie Brot, Milch, Fruchtsäfte, Gemüse und Obstvollkonserven oder Säuglingsnahrung, mit Vitaminen angereichert.

Spurenelemente sind anorganische Verbindungen, die gewöhnlich im Organismus als Bau- und Wirkstoffe dienen. Sie befinden sich im Erdboden, in den Gesteinen und zum Teil auch im Wasser und werden von den Pflanzen über die Wurzeln aufgenommen und in Form von Nahrung an tierische Organismen weitergegeben. Viele Spurenelemente sind für den menschlichen Organismus unentbehrlich und sind an wichtigen Vorgängen, z.B. am Stoffwechsel, der Regulation des pH-Wertes und des osmotischen Drucks und der Bildung einer Vielzahl von organischen Verbindungen im Körper, beteiligt. Die Folge eines Mangels an Spurenelementen sind schwere Erkrankungen des Organismus bis hin zum Tod. Deshalb werden Spurenelemente verstärkt verschiedenen Lebensmitteln beigefügt. Weiterhin ist die Einnahme von Präparaten mit Vitaminen und Spurenelementen in Form von Tabletten, Kapseln und Brausetabletten als Nahrungsergänzungsmittel oder bei höheren Konzentrationen der Vitamine und Spurenelemente als Arzneimittel weit verbreitet.

Pflanzenextrakte können aus Pflanzen verschiedener Art gewonnen werden. Diese Pflanzenextrakte sind ihrerseits reich an natürlichen Vitaminen. Darüber hinaus enthalten sie sekundäre Pflanzeninhaltsstoffe, die für eine Verwendung der Pflanzenextrakte als Nahrungsergänzungsmittel von Bedeutung sind. So schützen diese Inhaltsstoffe die Vitamine bei der Passage vom Mund durch den Magen bis hin zum Darm. Weiterhin fördern sie die Aufnahme der Vitamine im Dünndarm und steigern die antioxidative Wirkung, d.h. die Bindung von freien Radikalen, der Vitaminkombinationen. Diese steigernde Wirkung kann vor allem durch den Gehalt an Bioflavonoiden in Pflanzenextrakten erhalten werden. Des weiteren entfalten die sekundären Pflanzeninhaltsstoffe unter anderem anregende, vitalisierende und abwehrstärkende Wirkungen.

Die derzeit im Handel angebotenen Nahrungsergänzungsmittel bzw. Arzneimittel auf der Basis von Vitaminen, Spurenelementen oder Pflanzenextrakten lassen sich in zwei Kategorien einteilen. Zum einen sind Tabletten oder Kapseln erhältlich, die als Grundsubstanz synthetisch hergestellte Vitamine, Spurenelemente oder Pflanzenextrakte , die auf ein Trägermaterial, wie Dextrose, aufgezogen sind, enthalten. Diesen Grundsubstanzen werden zur Tablettierung übliche Tablettierungsstoffe, wie Stärke, beigefügt, und die Zusammensetzung wird gepreßt, um die gewünschte Tablettenform zu erhalten. Nahrungsergänzungsmittel in Form von Brausetabletten enthalten üblicherweise ebenfalls die Grundzusammensetzung aus Vitaminen, Spurenelementen und einen Träger. Zur sprudelnden Auflösung in Wasser enthalten Brausetabletten weiterhin Carbonate, wie Calciumcarbonat und Magnesiumcarbonat, Hydrogencarbonate, wie Kaliumhydrogencarbonat, und Zitronensäure.

Diese bisher üblichen Darreichungsformen von Nahrungsergänzungsmitteln und Arzneimitteln auf der Basis von Vitaminen, Spurenelementen oder Pflanzenextrakten weisen jedoch einige Nachteile auf. So ist die Einnahme von Tabletten für viele Verbraucher nicht attraktiv. Brausetabletten haben den Nachteil, daß sie zur Auflösung eine größere Menge einer Flüssigkeit, wie Leitungswasser benötigen, das nicht immer oder nicht in ausreichend guter Qualität vorhanden ist. Die Zusammensetzung der bisher verwendeten Nahrungsergänzungsmittel und Arzneimittel ist nicht geeignet, direkt auf die Zunge appliziert zu werden, um durch den Speichel gelöst zu werden, da sie leicht verklumpen und sich nicht sofort vollständig auflösen.

Zu bekannten Zusammensetzungen, die sich direkt durch Kontakt mit dem Speichel lösen, gehören Schleck- oder Eßbrausen. Diese Produkte bestehen hauptsächlich aus Zucker, Aromen und Farbstoffen. Die Schleck- oder Eßbrausen enthalten üblicherweise Lösehilfen, wie eine Kombination von organischen Säuren, z.B. Zitronensäure, mit Hydrogencarbonaten. Durch das Vorhandensein der organischen Säuren und Hydrogencarbonat kommt es zu einem brausenden Effekt auf der Zunge, der für den Zweck dieses Produktes als Genußmittel für Kinder zwar attraktiv ist, bei einer Verwendung als Arznei- oder Nahrungsergänzungsmittel jedoch nicht erwünscht ist.

Es ist daher die Aufgabe der vorliegende Erfindung, Vitaminpräparate in einer verbesserten Darreichungsform zur Verfügung zu stellen, die in der Form der Einnahme attraktiver und hygienischer gestaltet sind. Das Vitaminpräparat soll daher direkt in die Mundhöhle appliziert werden und durch den Speichel sofort gelöst werden können, ohne daß eine weitere Flüssigkeitszufuhr notwendig ist. Des weiteren sollen die Bestandteile des Vitaminpräparates homogen mischbar sein.

Diese Aufgabe wird erfindungsgemäß durch ein Vitaminpräparat gelöst, welches die folgenden Bestandteile umfaßt:
(a) eine Komponente aus (A) einem Trägermaterial aus mindestens einem Zucker oder einem Zuckerderivat in Form eines feinen Pulvers, auf das (B) mindestens ein Vitamin und/oder Spurenelement und/oder Pflanzenextrakt aufgebracht ist, und
(b) mindestens ein Bindemittel in kristalliner oder agglomerierter Form.

Die vorliegende Erfindung stellt weiterhin ein Pulver zur Verfügung, das dieses Vitaminpräparat enthält.

Ferner stellt die vorliegende Erfindung ein Verfahren zur Herstellung dieses Vitaminpräparates zur Verfügung, umfassend:
- das Aufziehen der Teilkomponente (B) auf das Trägermaterial (A) zur Herstellung der Komponente (a); und
- das Mischen der Komponente (a) mit dem Bindemittel (b).

Das erfindungsgemäße Vitaminpräparat enthält übliche Zusammensetzungen an Vitaminen und/oder Spurenelementen und/oder Pflanzenextrakten. Die verwendeten Vitamine sind üblicherweise und überwiegend wasserlösliche Vitamine und umfassen Vitamine der B-Gruppe, wie Vitamin B1, B2, B6 und B12, Vitamin C, Biotin, Folsäure und Niacin. Es können aber auch öllösliche Vitamine, wie Vitamin E oder Vitamin A, zum Einsatz kommen. Die wasserlöslichen Vitamine können sofort in den Organismus gelangen. Die öllöslichen Vitamine gelangen mit dem Speichel durch das Schlucken in den Magen und werden dort gelöst. Um die Verfügbarkeit der öllöslichen Vitamine zu erhöhen, werden diese vorzugsweise mit einer Ölkomponente in an sich bekannter Weise formuliert, bevor sie auf das Trägermaterial aufgezogen werden. Die Vitamine können synthetisch hergestellt werden oder auch aus natürlichen Quellen, wie pflanzlichen oder tierischen Organismen, gewonnen werden. Die Vitamine werden normalerweise miteinander kombiniert. In dieser Ausführungsform ist es bevorzugt, daß sie in dem erfindungsgemäßen Vitaminpräparat zielgruppengerecht kombiniert werden. Es kann aber auch nur ein Vitamin in dem erfindungsgemäßen Präparat enthalten sein. Dies ist üblicherweise der Fall, wenn das Präparat als Arzneimittel verwendet wird, um eine bestimme Vitaminmangelerscheinung zu behandeln.

Zusätzlich zu den Vitaminen kann das erfindungsgemäße Präparat auch mindestens ein Spurenelement beinhalten. Als Spurenelemente kommen Arsen, Brom, Cadmium, Chlor, Chrom, Cobalt, Fluor, Iod, Kupfer, Molybdän, Nickel, Niobium, Schwefel, Selen, Silicium und Zinn in Frage. Zu den in der vorliegenden Erfindung bevorzugt eingesetzten Spurenelementen zählen Fluor, Silicium, Iod, Selen, Chrom und Molybdän.

Als weiterer Bestandteil kann auch mindestens ein Pflanzenextrakt in dem erfindungsgemäßen Präparat enthalten sein. Beispiele für Pflanzenextrakte sind Hagebuttenextrakt, Citrusextrakt, beispielsweise mit Bioflavonoiden, Holunderbeerenextrakt, Lindenblütenextrakt, getrockneter Acerolasaft, Weizenextrakt, Extrakt aus der Alge Dunelia, Algenextrakt, z.B. aus Focus, Hefeextrakt oder Hefeautolysat, Selenhefe, Tomatenextrakt und Extrakte aus Matetee oder Grüntee. Zu den bevorzugt verwendeten natürlichen Pflanzenextrakten zählen Grüntee-Extrakt, Hagebuttenextrakt und Citrusextrakt.

Das erfindungsgemäße Präparat kann weiterhin andere Stoffe zur Nahrungsergänzung, wie Coenzyme, z.B. Coenzym Q10, und/ oder Gelee Royal, umfassen.

Die Kombination aus Vitaminen und/oder Spurenelementen und/oder Pflanzenextrakten ist in dem erfindungsgemäßen Vitaminpräparat üblicherweise in einer Menge von 5 bis 50 Gew.-%, vorzugsweise von 15 bis 30 Gew.-% enthalten. Wird diese Kombination hoch dosiert, ist das erfindungsgemäße Vitaminpräparat insbesondere als Arzneimittel geeignet. Die Vitamine und/oder Spurenelemente und/oder Pflanzenextrakte werden pro Dosiereinheit, d.h. pro Beutel, üblicherweise in der empfohlenen Tagesdosis, sofern bekannt, dosiert. Da die Haltbarkeit einiger Bestandteile dieser Vitamin/Spurenelemente/Pflanzenextraktekombination nicht sehr hoch ist, können diese oder alle Bestandteile auch überdosiert werden.

Das erfindungsgemäße Vitaminpräparat enthält weiterhin als Trägermaterial mindestens einen Zucker oder ein Zuckerderivat in Form eines feinen Pulvers, auf das die Vitamine und/oder Mineralstoffe und/oder Spurenelemente aufgebracht sind. Dieses Trägermaterial wird vorzugsweise aus Dextrose, Lactose, Maltose, Maltodextrin oder Mischungen davon ausgewählt. Besonders bevorzugt wird Dextrose und/oder Maltosedextrin eingesetzt. Das Trägermaterial besitzt eine relativ geringe Korngröße, die vorzugsweise nicht größer als 450 µm ist. Üblicherweise ist sie geringer als 175 µm. Es ist besonders bevorzugt, daß ca. 95% des Trägermaterials feiner sind als 150 µm und 100% feiner sind als 425 µm.

Üblicherweise liegt das Trägermaterial in einer Menge von 3 bis 70 Gew.-%, bezogen auf die Kombination von Vitaminen und/oder Spurenelementen und/oder Pflanzenextrakten, vor. Bevorzugt ist ein Anteil von 5-50 Gew.-%, bezogen auf die Kombination von Vitaminen und/oder Spurenelementen und/oder Pflanzenextrakten, im Präparat. Wenn der Anteil an Trägermaterial geringer ist als 3 Gew.-%, werden die Vitamine und/oder Spurenelemente und/oder Pflanzenextrakte, insbesondere die Vitamine, nicht ausreichend gebunden. Bei einem Einsatz von größeren Mengen als 70 Gew.-% des Trägermaterials, bezogen auf die Vitamine und/oder Spurenelemente und/oder Pflanzenextrakte, kommt es zu einer verminderten Rieselfähigkeit des Vitaminpräparates, da der Anteil an feinem Pulver im Vitaminpräparat zu hoch wird.

Weiterhin enthält das erfindungsgemäße Vitaminpräparat mindestens ein Bindemittel in kristalliner oder agglomerierter Form. Unter dem Begriff 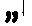 kristallin" wird im folgenden eine Molekülanordnung verstanden, die im Gegensatz zu amorphen Stoffen eine Kristallstruktur enthält. Als Bindemittel können beispielsweise agglomerierter Glucosesirup, sowie Kristallzucker, Maltodextrin, Saccharose, Lactobiose, Lactose und Fructose, aber auch Vitamin C, jeweils in kristalliner oder agglomerierter Form, verwendet werden. Wird Vitamin C als Bindemittel eingesetzt, enthält das erfindungsgemäße Vitaminpräparat zusätzlich zu Vitamin C mindestens ein weiteres Vitamin und/oder mindestens einen Pflanzenextrakt und/oder mindestens ein Spurenelement. Der Einsatz von agglomeriertem Glucosesirup ist aufgrund seiner Verfügbarkeit und Erscheinung bevorzugt. Das Bindemittel kann in einer weiteren bevorzugten Ausführungsform Kristallzucker sein, der zur Aufrauhung der Oberfläche gespalten oder gemahlen sein kann. Es ist auch möglich, die vorstehend genannten Bindemittel in Kombination zu verwenden. In diesem Fall ist der Einsatz von agglomeriertem Glucosesirup oder Vitamin C in Verbindung mit Kristallzucker bevorzugt.

Das Bindemittel besitzt eine wesentlich gröbere Körnung als das Trägermaterial und ergibt somit ein Endprodukt, das bei der Herstellung aufgrund seiner Fließfähigkeit und verminderten Staubentwicklung sehr leicht gehandhabt werden kann. Es besitzt üblicherweise eine Korngröße von 500 bis 100 µm, vorzugsweise 450 bis 150 µm. Bei der Verwendung von agglomeriertem Glucosesirup ist es bevorzugt, daß mindestens 95% Rückstand auf einem 40 µm Sieb, ca. 60% Rückstand auf einem 200 µm Sieb und maximal 5% Rückstand auf einem 500 µm Sieb erhalten werden. Wird dagegen Kristallzucker oder kristalline Fructose verwendet, besitzen diese eine geringere Korngröße, die jedoch immer noch wesentlich größer ist als die des Trägermaterials. Üblicherweise werden Kristallzucker oder Fructose mit einer Größe von 80 µm bis 350 µm, vorzugsweise von 100 µm bis 315 µm, für 90% der Kristallmasse verwendet. Das Bindemittel dient hauptsächlich dazu, dem Produkt gute Rieseleigenschaften zu verleihen. Durch die agglomerierte bzw. kristalline Form des Bindemittels ergibt sich auch ein sehr gutes Fließverhalten für die Herstellung, was insbesondere bei der Mischung und der Abpackung vorteilhaft ist. Des weiteren läßt sich durch den sehr kleinen Anteil an feinen Partikeln unter 30 µm eine starke Staubentwicklung bei der Herstellung und Mischung des Endproduktes vermeiden.

Durch die agglomerierte bzw. kristalline Form des Bindemittels kommt es zu einem sehr schnellen Auflösen beim Kontakt mit einer geringen Flüssigkeitsmenge, z.B. Speichel. Weiterhin dispergiert das Vitaminpräparat ausgezeichnet in Flüssigkeiten. Es kann ein Auflösen des Vitaminpräparates ohne jegliche Verklumpungen erreicht werden, und die Vitamine liegen in einer vom Körper leicht aufnehmbaren, d.h. in einer sofort verfügbaren Form, vor.

Um die vorstehend beschriebene Rieselfähigkeit, die das Bindemittel dem erfindungsgemäßen Vitaminpräparat in Pulverform verleiht, in vollem Umfang zu erhalten, ist es von Vorteil, daß der Anteil des Bindemittels im Vitaminpräparat in dieser Ausführungsform sehr hoch ist. Da das erfindungsgemäße Vitaminpräparat in Pulverform angeboten wird, ist es bevorzugt, daß die Menge des Bindemittels mindestens doppelt so hoch ist wie die Gesamtmenge der Vitamine und/oder Spurenelemente und/oder Pflanzenextrakte. Üblicherweise wird für das erfindungsgemäße Vitaminpräparat in Pulverform eine Menge, die dem Drei- bis Fünffachen der Gesamtmenge der Vitamine und/oder Spurenelemente und/oder Pflanzenextrakte entspricht, verwendet. Wenn gewünscht, kann die Menge des Bindemittels jedoch auch weniger als das Doppelte der Gesamtmenge der Vitamine und/oder Spurenelemente und/oder Pflanzenextrakte betragen. Dies ist insbesondere bei einer Verwendung von Vitamin C als Bindemittel bevorzugt. Wenn in dem Vitaminpräparat in Pulverform mit einer 600 mg Dosierung die Summe an Vitaminen und/oder Spurenelementen und/oder Pflanzenextrakten ca. 80 bis 250 mg beträgt, und das Trägermaterial 3 bis 70 Gew.-%, bezogen auf diese Summe, ausmacht, wird die restliche Menge durch das Bindemittel in agglomerierter bzw. kristalliner Form ausgeglichen.

Wenn die Süßkraft des Bindemittels nicht ausreicht, um dem Vitaminpräparat einen angenehmen Geschmack zu verleihen, können weitere Süßungsmittel, wie künstliche Süßungsmittel, enthalten sein. Beispiele für solche Süßungsmittel sind Aspartam, Acesulfam K, Saccharin und Cyclamat, sowie Kombinationen davon. Bevorzugt sind Acesulfam K und Aspartam und Mischungen davon. Das künstliche Süßungsmittel kann in dem erfindungsgemäßen Präparat in einer Menge von 0,1 bis 5,0 Gew.-%, vorzugsweise 0,1 bis 0,7 Gew.-%, enthalten sein. In einer 600 mg Portion können demzufolge 0,4 bis 30 mg, vorzugsweise 0,4 bis 4 mg, künstliches Süßungsmittel enthalten sein.

Wie vorstehend erwähnt, liegt das erfindungsgemäße Vitaminpräparat in Pulverform vor. Zur Herstellung des Präparates in Pulverform werden die einzelnen Bestandteile gemischt. Dabei werden zuerst die Vitamine und/oder Spurenelemente und/oder Pflanzenextrakte auf das Trägermaterial, d.h. den Zucker oder das Zuckerderivat in Form eines feinen Pulvers, aufgezogen. Die so erhaltene Grundzusammensetzung wird mit dem Bindemittel in agglomerierter oder kristalliner Form gemischt. Gegebenenfalls kann mindestens ein weiterer Pflanzenextrakt vor, nach oder gleichzeitig mit der Zumischung des Bindemittels in agglomerierter oder kristalliner Form zugemischt werden. Wenn die Vitamine und/oder Spurenelemente und/oder Pflanzenextrakte gleichzeitig mit dem Trägermaterial und dem Bindemittel gemischt werden, kann kein homogenes Pulver erhalten werden, da es nicht möglich ist, diese Bestandteile zu binden. Das erfindungsgemäße Vitaminpräparat kann in Form von Tagesdosierungen portioniert und abgepackt werden.

Das erfindungsgemäße Vitaminpräparat kann auf verschiedene Weise angewendet werden. Vorteilhafterweise kann das Vitaminpräparat direkt auf die Zunge gestreut werden und löst sich bei Kontakt mit dem Speichel auf Dadurch ist es jederzeit und überall verzehrbar. Das Vitaminpräparat kann jedoch auch in flüssiger Form, d.h. durch Lösen des Präparates in einem kalten Getränk, z.B. Eistee, Saft, Cola oder Mineralwasser, eingenommen werden. Dabei wird das Pulver zu dem Getränk gegeben und ist nach wenigen Sekunden ohne umzurühren trinkbereit. Es ist besonders bevorzugt, das Vitaminpräparat in Tagesdosierungen, beispielsweise in einer Menge von 0,5 g bis 1,0 g, anzubieten. Dadurch kann der Verbraucher durch einmalige Einnahme des Vitaminpräparates seinen täglichen Vitaminbedarf decken.

Vorteilhafterweise wird das erfindungsgemäße Vitaminpräparat in verschiedenen Zusammensetzungen und Dosierungen angeboten. Durch die Variation der Mengen der eingesetzten Vitamine und/oder Spurenelemente und/oder Pflanzenextrakte und der speziellen Auswahl von einzelnen Vitaminen und/oder Spurenelementen und/oder Pflanzenextrakten kann das Präparat zielgruppengerecht zur Verfügung gestellt werden. Geeignete Zusammensetzungen beinhalten beispielsweise Zusammensetzungen bei einer einseitigen Ernährung (Basisdosierung), Zusammensetzungen für Junioren, Frauen verschiedener Altersgruppen, Männer, Senioren, Sportler, Vielflieger, Raucher und Diabetiker.

Die vorliegende Erfindung wird anhand der folgenden Beispiele näher erläutert:

### Beispiel 1

### Vitaminpräparat bei einseitiger Ernährung:

Die Bestandteile für eine Zubereitung des erfindungsgemäßen Vitaminpräparates sind in den Mengen für eine 0,6 g Dosierung wie folgt angegeben.

| | | |
|---|---|---|
| 1. | Vitamine/Spurenelemente | |
| | Vitamin E | 10 mg = 100% der nach der DGE empfohlenen Tagesdosis |
| | Vitamin C | 60 mg = 100% |
| | Vitamin B1 | 1,4 mg = 100% |
| | Vitamin B2 | 0,8 mg = 50% |
| | Vitamin B6 | 2,0 mg = 100% |
| | Niacin | 5 mg = 27% |
| | Vitamin B12 | 1 µg = 100% |
| | Folsäure | 0,2 mg = 100% |
| | Biotin | 0,15 mg = 100% |
| | Selen | 20 µg (DGE Schätzwert: 20 - 100 µg täglich) |
| | Fluor | 0,4 mg = 1,0 mg NaF (DGE-Empfehlung 1 mg/Tag) |
| | Silicium | 1 µg (= 2,5 µg SiO₂) |
| | Hilfsstoffe, die bei der Vitaminherstellung verwendet werden | 20,028 mg |
| 2. | Pflanzenextrakte und andere Bestandteile | |
| | Gelee Royal | 1 mg |
| | Coenzym Q10 | 5 mg |
| | Grüntee-Extrakt | 5 mg |
| | Citrusextrakt (mit Bioflavonoiden) | 5 mg |
| | Hagebuttenextrakt | 10 mg |
| | Holunderbeerenextrakt | 10 mg |
| | Mate-Extrakt | 5 mg |
| | Acerolasaft, getrocknet | 5 mg |
| | Lindenblütenextrakt | 5 mg |
| 3. | Dextrose (25% von der Vitamin/ Spurenelementekombination) | 22,500 mg |
| 4. | Kristallzucker | 426,500 mg |
| | | 600,000 mg (Tagesdosis) |

### Beispiel 2

### Vitaminpräparat für Senioren

Die Bestandteile für eine Zubereitung des erfindungsgemäßen Vitaminpräparates sind in den Mengen für eine 0,6 g Dosierung wie folgt angegeben.

| | | |
|---|---|---|
| 1. | Vitamine/Spurenelemente | |
| | Vitamin C | 60 mg = 100% der nach der DGE empfohlenen Tagesdosis |
| | Vitamin E | 10 mg = 100% |
| | Vitamin B1 | 2,8 mg = 200% |
| | Vitamin B2 | 0,8 mg = 50% |
| | Vitamin B6 | 4,0 mg = 200% |
| | Niacin | 5 mg = 27% |
| | Vitamin B12 | 3 µg = 300% |
| | Selen | 20 mg (DGE-Schätzwert 20 - 100 µg täglich) |
| | Chrom | 25 µg (DGE-Schätzwert 50 - 200 µg täglich) |
| | Fluor | 0,4 mg = 1,0 mg Natriumfluorid |
| | Silicium | 1 µg = 2,5 µg Siliciumdioxid |
| | Hilfsstoffe, die bei der Vitaminherstellung verwendet werden | 20,151 mg |
| 2. | Pflanzenextrakte und andere Bestandteile | |
| | Coenzym Q10 | 20 mg |
| | Gelee Royal | 1 mg |
| | Citrusextrakt (mit Bioflavonoiden) | 5 mg |
| | Lindenblütenextrakt | 5 mg |
| | Holunderbeerenextrakt | 10 mg |
| | Hagebuttenextrakt | 10 mg |
| | Acerolasaft, getrocknet | 5 mg |
| 3. | Dextrose (25% von der Vitamin/ Spurenelementekombination | 28,800 mg |
| 4. | Kristallzucker | 412,0 mg |
| | | 600,00 mg (Tagesdosis) |

### Beispiel 3

### Vitaminpräparat bei Strahlenbelastung (Vielflieger, Sonne usw.)

Die Bestandteile für eine Zubereitung des erfindungsgemäßen Vitaminpräparates sind in den Mengen für eine 0,5 g Dosierung wie folgt angegeben.

| | | |
|---|---|---|
| 1. | Vitamine/Spurenelemente | |
| | Vitamin E | 36 mg = 300% (der nach der DGE empfohlenen Tagesdosis) |
| | Vitamin C | 150 mg = 200% |
| | Selen | 100 µg (DGE-Schätzwert 200-100 µg täglich) |
| 2. | Pflanzenextrakt | |
| | Grüntee-Extrakt | 50 mg |
| 3. | Dextrose (5% von der Vitamin/ Mineralstoff/Spurenelementekombination) | 11,80 mg |
| 4. | Glucosesirup in agglo-merierter Form | 250,10 mg |
| 5. | Aspartam | 1 mg |
| | Acesulfam | 1 mg |
| | | 500,00 mg (Tagesdosis) |

### Beispiel 4

### Vitaminpräparat als Arzneimittel bei Vitamin-E-Mangel

Die Bestandteile für eine Zubereitung des erfindungsgemäßen Vitaminpräparates sind in den Mengen für eine 0,8 g Dosierung wie folgt angegeben:

| | | |
|---|---|---|
| 1. | Vitamin E | 400 mg |
| 2. | Dextrose (35% von der Vitamin/ Mineralstoff/Spurenelementekombination | 175 mg |
| 3. | Vitamin C in kristalliner Form | 125 mg |
| | Kristallzucker | 100 mg |
| | | 800 mg (Tagesdosis) |

### Beispiel 5

### Herstellung des Vitaminpräparates in Pulverform

Alle Bestandteile des Vitaminpräparates gemäß einem der Beispiele 1 bis 4 wurden in den entsprechenden Mengen abgewogen, um 5 kg des Vitaminpräparates herzustellen. Die Vitamine bzw. Spurenelemente wurden danach auf Dextrose aufgezogen. Die so erhaltene Zusammensetzung wurde dann mit der entsprechenden Menge an Bindemittel und gegebenfalls Pflanzenextrakt gemischt, wenn angegeben unter Zugabe eines Süßungsmittels, bis ein homogenes Pulver erhalten wurde.

Das erhaltene Vitaminpräparat wurde in der jeweils angegebenen Menge in Beutel gefüllt und stellt die Tagesdosierung der jeweiligen Zielgruppe dar.

Zur Einnahme wurde das Vitaminpräparat aus dem Beutel auf die Zunge gestreut, wobei es zum sofortigen vollständigen Auflösen des Präparates kam.

### Vergleichsbeispiel 1

Die in Beispiel 1 angegebenen Bestandteile, außer dem Bindemittel, wurden gemischt und in Beutel abgefüllt. Das so erhaltene Vitaminpräparat neigte zu Verklumpungen und ließ sich nur schlecht aus dem Beutel auf die Zunge applizieren. Wenn es auf die Zunge gegeben wurde, löste es sich nur unzureichend und auch dann nur unter Klumpenbildung.

Die Löslichkeit in größeren Flüssigkeitsmengen, etwa 0,2 l, war ebenfalls schlecht.

### Vergleichsbeispiel 2

Die in Beispiel 1 angegebenen Bestandteile wurden gemeinsam gemischt. Trotz langer Mischdauer konnten die Vitamine nicht mit dem Trägermaterial und dem Bindemittel gebunden werden, so daß kein homogenes Pulver entstand.

## Patentansprüche

1. Vitaminpräparat, umfassend:
(a) eine Komponente aus (A) einem Trägematerial aus mindestens einem Zucker oder einem Zuckerderivat in Form eines feinen Pulvers, auf das (B) mindestens ein Vitamin und/oder Spurenelement und/oder Pflanzenextrakt aufgebracht ist, und
(b) mindestens ein Bindemittel in kristalliner oder agglomerierter Form.

2. Vitaminpräparat nach Anspruch 1, **dadurch gekennzeichnet,** daß das Trägermaterial (A) aus Dextrose, Lactose, Maltose, Maltodextrin oder Mischungen davon gewählt wird.

3. Vitaminpräparat nach Anspruch 1 oder 2, **dadurch gekennzeichnet,** daß das Trägermaterial (A) 3-70%, bezogen auf die Gesamtmenge der Teilkomponente (B), ausmacht.

4. Vitaminpräparat nach Anspruch 1 bis 3, **dadurch gekennzeichnet,** daß das Bindemittel (b) agglomerierter Glucosesirup und/oder Kristallzucker ist.

5. Vitaminpräparat nach einem der Ansprüche 1 bis 4, **dadurch gekennzeichnet,** daß die Menge des Bindemittels (b) mindestens doppelt so hoch ist wie die Gesamtmenge der Teilkomponente (B).

6. Pulver, enthaltend das Vitaminpräparat nach einem der Ansprüche 1 bis 5.

7. Verfahren zur Herstellung eines Vitaminpräparates nach einem der Ansprüche 1 bis 5, umfassend:
- das Aufziehen der Teilkomponente (B) auf das Trägermaterial (A) zur Herstellung der Komponente (a); und
- das Mischen der Komponente (a) mit dem Bindemittel (b).

8. Verfahren nach Anspruch 7, **dadurch gekennzeichnet,** daß es als weiteren Schritt das Zumischen mindestens eines nicht auf das Trägermaterial (A) aufgezogenen Pflanzenextrakts umfaßt.

9. Verwendung des Vitaminpräparates nach einem der Ansprüche 1 bis 5 als Nahrungsergänzungsmittel und/oder Arzneimittel.

10. Verwendung des Pulvers nach Anspruch 6 als Nahrungsergänzungsmittel und/oder Arzneimittel.
